# EUROPEAN PATENT APPLICATION

(11) **EP 4 613 263 A1**
(43) Date of publication of application: **10.09.2025**
(21) Application number: 23885681.9
(22) Date of filing: 27.10.2023
(51) Int. Cl.: A61K 8/81, A45D 29/00, A61K 8/35, A61Q 3/02

(54) **PHOTOCURABLE COMPOSITION FOR NAILS OR ARTIFICIAL NAILS**

(30) Priority: 04.11.2022 JP 2022177271
(71) Applicant: ThreeBond Co., Ltd., Tokyo 192-0398 (JP)
(72) Inventor: MORIKAWA, Yumi, Hachioji-shi, Tokyo 192-0398 (JP); HARADA, Natsumi, Hachioji-shi, Tokyo 192-0398 (JP)
(74) Representative: Crow, Martin
(86) International application number: PCT/JP2023/038946
(87) International publication number: WO 2024/095925

(57) **Abstract**

To provide a photocurable composition for a nail or an artificial nail, which can allow for formation of a cured product having excellent wear resistance and having high gloss properties.

A photocurable composition for a nail or an artificial nail, comprising the following components (A) to (E), in which, in a case where the component (C) comprises a component (c-1), a monofunctional (meth)acrylate is not contained as the component (D) or a content of a monofunctional (meth)acrylate as the component (D) is 50% by mass or less based on a total of the component (D):
component (A): a urethane-modified (meth)acrylate oligomer;
component (B): a (meth)acrylate having a bisphenol backbone;
component (C): one or more (meth)acrylates selected from the group consisting of a component (c-1) and a component (c-2) (except for the component (A) and the component (B)):
component (c-1): a (meth)acrylate having an alicyclic hydrocarbon backbone; and
component (c-2): a (meth)acrylate having an isocyanurate backbone;

component (D): a (meth)acrylate except for components (A) to (C); and
component (E): a photoinitiator.

## Description

### TACHNICAL FIELD

The present invention relates to a photocurable composition for a nail or an artificial nail.

### BACKGROUND ART

So-called gel nails are obtained by applying a photocurable composition directly or indirectly to a human nail and curing it by irradiation with light, thereby obtaining a nail decorative film that has high adhesion to the nail and has a beautiful luster. Japanese Patent Laid-Open No. 2017-210475 discloses a photocurable artificial nail composition having luster, the composition containing a urethane (meth)acrylate oligomer, a (meth)acrylic monomer, a polythiol compound and a photopolymerization initiator. According to the Literature, disclosed is the following: the polythiol compound is added to the (meth)acrylic monomer to obtain enhancement in photocurability, accordingly hardly affected by curing inhibition due to oxygen even at an interface in contact with oxygen, thereby obtaining a gel nail enhanced in surface curability.

### SUMMARY OF INVENTION

However, a cured product of the composition described in Japanese Patent Laid-Open No. 2017-210475 has been sometimes lost in gloss properties because a surface thereof is finely scratched due to external friction caused by rubbing a waste cloth, cotton, or the like. On the other hand, a gel nail containing no polythiol compound is easily affected by curing inhibition due to oxygen, and an uncured component remains to sometimes cause deterioration in surface curability. In such a case, it is necessary to wipe off such an uncured component on a surface with a solvent by use of a waste cloth, cotton, or the like, and thus a cured product surface is affected, for example, scratched, and/or lost in gloss and then delustered. As described above, conventionally, a cured product surface has been easily finely scratched due to external friction or the like by a waste cloth, cotton, or the like, and has encountered a difficulty in expressing sufficient gloss.

The present invention has been made in view of the above circumstances, and an object thereof is to provide a photocurable resin composition for a nail or an artificial nail, which can allow for formation of a cured product having excellent wear resistance and having high gloss properties.

### Solution to Problem

The gist of the present invention is then described.
[1] A photocurable composition for a nail or an artificial nail, comprising the following components (A) to (E), wherein, in a case where the component (C) comprises a component (c-1), a monofunctional (meth)acrylate is not contained as the component (D) or a content of a monofunctional (meth)acrylate as the component (D) is 50% by mass or less based on a total of the component (D):
   component (A): a urethane-modified (meth)acrylate oligomer;
   component (B): a (meth)acrylate having a bisphenol backbone;
   component (C): one or more (meth)acrylates selected from the group consisting of a component (c-1) and a component (c-2) (except for the component (A) and the component (B)):
      component (c-1): a (meth)acrylate having an alicyclic hydrocarbon backbone; and
      component (c-2): a (meth)acrylate having an isocyanurate backbone;
   component (D): a (meth)acrylate except for components (A) to (C); and
   component (E): a photoinitiator.
[2] The photocurable composition for a nail or an artificial nail according to [1], comprising 30 parts by mass or more and 90 parts by mass or less of the component (B) based on 100 parts by mass of the component (A).
[3] The photocurable composition for a nail or an artificial nail according to [1] or [2], wherein the component (D) is one or more selected from the group consisting of a monofunctional (meth)acrylate, a difunctional (meth)acrylate and a trifunctional (meth)acrylate.
[4] The photocurable composition for a nail or an artificial nail according to any of [1] to [3], further comprising an ultraviolet absorber.
[5] The photocurable composition for a nail or an artificial nail according to [4], wherein the ultraviolet absorber is a hydroxybenzophenone derivative.
[6] The photocurable composition for a nail or an artificial nail according to any of [1] to [5], wherein the photocurable composition for a nail or an artificial nail is for topcoat layer formation.

### DESCRIPTION OF EMBODIMENTS

The detail of the present invention is then described. The present invention is not limited only to the following embodiments, and can be variously modified within the claims. Embodiments described herein can be arbitrarily combined to provide any other embodiment.

Throughout the specification, a singular expression is to be understood to also encompass its plural concept, unless particularly mentioned. Accordingly, a singular article (for example, "a", "an", and "the" in the case of English) is to be understood to also encompass its plural concept, unless particularly mentioned. The terms used herein are each to be understood to be used in the meaning usually in the art, unless particularly mentioned. Accordingly, all the technical terms and scientific term used herein have the same meanings as those usually understood by those skilled in the art to which the present invention belongs, unless otherwise defined. If there is an inconsistency, the present specification (including definitions) is prioritized.

Herein, "X to Y" is used to include numerical values (X and Y) before and after "to" as a lower limit value and an upper limit value, and means "X or more and Y or less". The "concentration" (%) is, unless particularly described, represented as a mass concentration (% by mass), and the "ratio" is a mass ratio, unless particularly described. Unless particularly noted, operations, and measurement of physical properties and the like are carried out in conditions of room temperature (20 to 25°C) and a relative humidity of 40 to 55% RH. In addition, the "A and/or B" is meant to include each of A and B, and a combination thereof.

One embodiment of the present invention is a photocurable resin composition for a nail or an artificial nail, comprising the following components (A) to (E):
component (A): a urethane-modified (meth)acrylate oligomer;
component (B): a (meth)acrylate having a bisphenol backbone;
component (C): one or more (meth)acrylates selected from the group consisting of a component (c-1) and a component (c-2) (except for the component (A) and the component (B)):
   component (c-1): a (meth)acrylate having an alicyclic hydrocarbon backbone; and
   component (c-2): a (meth)acrylate having an isocyanurate backbone;
   component (D): a (meth)acrylate except for components (A) to (C); and
   component (E): a photoinitiator;
in which, in a case where the component (C) comprises a component (c-1), a monofunctional (meth)acrylate is not contained as the component (D) or more than 0 and 50% by mass or less of a monofunctional (meth)acrylate as the component (D) is contained based on the total of the component (D). According to the photocurable resin composition for a nail or an artificial nail, having such a configuration, a cured product having excellent wear resistance and having high gloss properties can be formed. Accordingly, according to a photocurable resin composition for a nail or an artificial nail according to one embodiment of the present invention, no scratch can be caused on a surface even by performing wiping off after photocuring, and excellent gloss can be expressed. According to the photocurable composition of the present invention, a cured product having excellent luster can be obtained even by performing wiping off after photocuring. In the present invention, the "having gloss" means a glossiness of 85% or more in measurement described below, whereas the "luster" means a state where, when a surface is visually confirmed in many directions, the surface can be confirmed to be flat and smooth even in any direction and can be confirmed have gloss even in any direction.

### <Component (A)>

The photocurable composition of the present invention comprises a urethane-modified (meth)acrylate oligomer as the component (A). Hereinafter, "acryloyl" and "methacryloyl" are collectively called "(meth)acryloyl", and accordingly, the term "(meth)acryloyl group" encompasses both an acryloyl group (H₂C=CH-C(=O)-) and a methacryloyl group (H₂C=C(CH₃)-C(=O)-). A compound having a (meth)acryloyl group is also called (meth)acrylate. The term "(meth)acrylate" encompasses both acrylate and methacrylate, and the term "(meth)acrylic" encompasses both acrylic and methacrylic. The (meth)acryloyl group may be a mode in which the (meth)acryloyl group is in the form of a (meth)acryloyloxy group.

The urethane-modified (meth)acrylate oligomer is a (meth)acrylate oligomer having a urethane bond, and any oligomer having one or more urethane bonds and one or more (meth)acryloyl groups can be used. A urethane (meth)acrylate oligomer is added, to result in the effects of an enhancement in adhesiveness to a nail or an artificial nail and enhancements in curability and strength of a photocurable composition (coating film). The "oligomer" refers to a polymer in which two to several tens of repeating units (monomer units) (including a repeating unit other than a (meth)acrylate monomer) are repeated.

The component (A) is not particularly limited as long as it is an oligomer having one or more urethane bonds and one or more (meth)acryloyl groups, and preferably has 2 to 6 (meth)acryloyl groups, further preferably has 2 to 5 (meth)acryloyl groups, particularly preferably 2 to 3 (meth)acryloyl groups, most preferably 2 (meth)acryloyl groups. The (meth)acryloyl group of the component (A) is preferably an acryloyl group. The oligomer may further have, in addition to such urethane bonds and (meth)acryloyl groups, other functional group(s) such as a carboxyl group, a phosphoric acid group, and/or a hydroxyl group.

The weight average molecular weight of the oligomer of the component (A) is preferably 1,000 to 100,000, more preferably 1,200 to 30,000, further preferably 1,500 to 20,000, particularly preferably 2,000 to 15,000, most preferably 3,000 to 10,000. Such a range can not only allow a viscosity for favorable workability to be retained, but also improve curability of a cured product. Herein, the value adopted as the weight average molecular weight is obtained by measurement by gel permeation chromatography (GPC) with polystyrene as a standard substance.

The component (A) is most preferably a urethane-modified acrylate oligomer having a weight average molecular weight of 3,000 to 6,000 and containing 2 to 5 acryloyl groups per molecule from the viewpoint of an enhancement in durability and from the viewpoint of favorable photocurability. In one embodiment, the component (A) contains 2 to 5 (preferably 2 to 4, more preferably 2 to 3, particularly preferably 2) (meth)acryloyl groups per molecule, and has a weight average molecular weight of 3,000 to 6,000 (preferably 3,000 to 5,000, more preferably 3,200 to 5,500, particularly preferably 3,300 to 4,000). Such a configuration can allow for rapid curing without heat generation during photocuring. Furthermore, no tackiness remains and also gloss (in particular, luster) is expressed on a surface of a cured product.

The urethane-modified (meth)acrylate oligomer may be synthesized by a known method, and there is known a synthesis in which a urethane bond is formed from a polyol and a polyisocyanate, and a compound having a hydroxyl group and a (meth)acryloyl group in its molecule or (meth)acrylic acid is added to the remaining (unreacted) isocyanate group.

Examples of the polyol include a polyol compound having 2 or more hydroxyl groups in its molecule, and specific examples include polyether polyol, polyester polyol, caprolactone diol, bisphenol polyol, polyisoprene polyol, hydrogenated polyisoprene polyol, polybutadiene polyol, hydrogenated polybutadiene polyol, castor oil polyol, and polycarbonate diol. In particular, polyether polyol (preferably polyether diol) is preferred for introduction of a polyether backbone from the viewpoint of expression of glossiness. These may be used singly or in combinations of a plurality thereof.

Examples of the polyisocyanate include a compound having 2 or more isocyanate groups in its molecule, and specific examples include an aromatic polyisocyanate, an alicyclic polyisocyanate, and an aliphatic polyisocyanate. Examples of the aromatic polyisocyanate include 2,4-tolylene diisocyanate, 2,6-tolylene diisocyanate, 1,3-xylylene diisocyanate, 1,4-xylylene diisocyanate, tetramethylxylylene diisocyanate, diphenylmethane diisocyanate, naphthalene-1,5- diisocyanate, and triphenylmethane triisocyanate. Examples of the alicyclic polyisocyanate include isophorone diisocyanate, bis(4-isocyanatocyclohexyl)methane, 1,3-bis(isocyanatomethyl)cyclohexane, 1,4-bis(isocyanatomethyl)cyclohexane, norbornane diisocyanate, and bicycloheptane triisocyanate. Examples of the aliphatic polyisocyanate include hexamethylene diisocyanate, 1,3,6-hexamethylene triisocyanate, and 1,6,11-undeca triisocyanate. In particular, diisocyanate such as isophorone diisocyanate or hexamethylene diisocyanate is preferred.

Examples of the compound having a hydroxyl group and a (meth)acryloyl group include mono(meth)acrylates of dihydric alcohols such as ethylene glycol, propylene glycol, 1,3-propanediol, 1,3-butanediol, 1,4-butanediol, and polyethylene glycol, and mono(meth)acrylates or di(meth)acrylates of trihydric alcohols such as trimethylolethane, trimethylolpropane, and glycerin. These may be used singly or in combinations of a plurality thereof.

The component (A) used here is preferably a urethane (meth)acrylate oligomer of a polyether backbone, more preferably a difunctional urethane (meth)acrylate oligomer of a polyether backbone. Herein, other urethane (meth)acrylate oligomer, for example, a urethane (meth)acrylate oligomer of a polyester backbone, a urethane (meth)acrylate oligomer of a polycaprolactone backbone, a urethane (meth)acrylate oligomer of a polycarbonate backbone, or the like can also be used in combination.

In one embodiment, the component (A) is a compound represented by the following chemical formula (a-1): 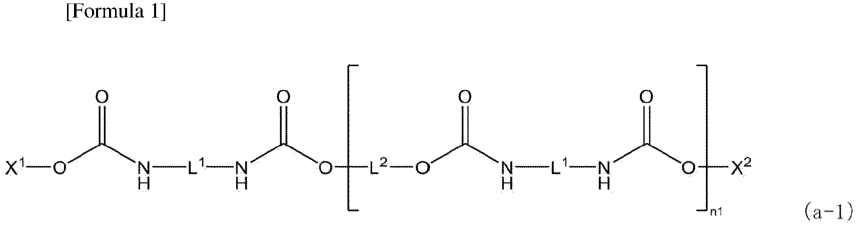 wherein,
L¹ is an alkylene group, a cycloalkylene group, an arylene group, or a group as a combination thereof,
L² is an alkylene group, an arylene group, a polyoxyalkylene group (*-[A¹-O]ₙ₂-A¹-*), a poly(oxycarbonylalkylene) group, a poly(oxycarbonyloxyalkylene) group, or a combination thereof,
   wherein A¹ is an ethylene group, a propylene group, a trimethylene group, or a tetramethylene group, and n2 is an integer of 0 to 100,
X¹ and X² are each independently a structure represented by the following: 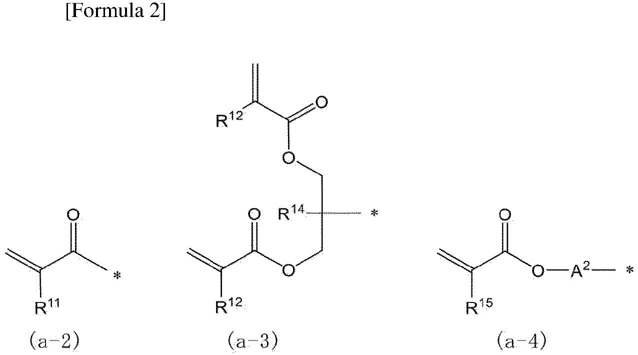
wherein,
   R¹¹, R¹² and R¹⁵ are each independently a hydrogen atom or a methyl group,
   R¹⁴ is a hydrogen atom, a methyl group, or an ethyl group, and
   A² is an ethylene group, a propylene group, a trimethylene group, or a tetramethylene group; and
n¹ is an integer of 1 to 100.

In one embodiment, L² in the chemical formula (a-1) contains a polyoxyalkylene group. In a case where the component (A) contains a molecule of such a structure, a cured product obtained by photocuring the photocurable composition can have gloss on a surface. In one embodiment, L² in the chemical formula (a-1) contains only a polyoxyalkylene group.

Herein, * represents a binding point with another atom or atom group.

Herein, the "alkylene group" refers to a substituted or unsubstituted alkylene group having 1 to 20 carbon atoms, 1 to 15 carbon atoms, 1 to 10 carbon atoms, 2 to 8 carbon atoms, or 2 to 4 carbon atoms. Examples of the alkylene group include a methylene group, an ethylene group, a propylene group, a trimethylene group, a tetramethylene group, a pentamethylene group, and a hexamethylene group, but not limited thereto.

Herein, the "oxyalkylene group" is a group represented by formula (a-5):

*-O-A³-* Formula (a-5)

wherein A³ is an alkylene group. Examples of the oxyalkylene group include an oxyethylene group, an oxypropylene group, an oxytrimethylene group, and an oxytetramethylene group, but not limited thereto.

Herein, the "arylene group" refers to a substituted or unsubstituted arylene group having 6 to 30 carbon atoms, 6 to 20 carbon atoms, 6 to 15 carbon atoms, or 6 to 10 carbon atoms. Examples of the arylene group include a phenylene group, a toluidine-diyl group, a xylene-diyl group, and naphthylene group, but not limited thereto.

Herein, the "substituted" refers to a hydrogen atom being substituted with any other atom or a substituent. Examples of such an atom or substituent include, unless especially defined, a halogen atom; an alkyl group having 1 to 20 carbon atoms, 1 to 10 carbon atoms, or 1 to 6 carbon atoms; an alkoxy group having 1 to 20 carbon atoms, 1 to 10 carbon atoms, or 1 to 6 carbon atoms; an aryl group having 6 to 30 carbon atoms, 6 to 20 carbon atoms, 6 to 15 carbon atoms, or 6 to 10 carbon atoms; or any combination thereof, but not limited thereto.

Specific examples of a commercially available product include Art Resin series, such as UN-6303, KY-11, UN-904, UN-6200, UN-6207, UN-6304, UN-6305, UN-6306, UN-6307, and UN-6060S manufactured by Negami Chemical Industrial Co., Ltd.; AH-600, AT-600, UA-306H, UA-510H, and UF-8001G manufactured by Kyoeisha Chemical Co., Ltd.; SUA-008 and SUA-023 manufactured by Asia Industry Co., Ltd.; and SHIKOH (registered trademark) series, such as UV-3310B and UV-6640B manufactured by Mitsubishi Chemical Corporation, but not limited thereto. The component (A) is not limited thereto, and two or more kinds thereof can also be used in combination. In a case where such two or more components (A) are used, the total content thereof is intended to be the content of the component (A).

### <Component (B)>

The photocurable composition of the present invention comprises a (meth)acrylate having a bisphenol backbone as the component (B). Herein, the component (B) is a component other than the component (A). The component (B) is preferably a (meth)acrylate monomer having a bisphenol backbone, more preferably one in which 2 or more (meth)acryloyl groups are bound to a bisphenol backbone directly or via a divalent linking group. The bisphenol backbone here refers to, for example, a bisphenol A type, bisphenol F type, or a novolac phenol type backbone. The component (B) can be synthesized by applying (meth)acrylic acid to a polyol having a bisphenol backbone or an epoxy resin having a bisphenol backbone, for addition reaction or ring-opening polymerization, but not limited thereto. In the present invention, the component (B) is preferably a bisphenol A type from the viewpoint of enhancements in strength and curability of a cured product.

The component (B) is not particularly limited in terms of the number of (meth)acryloyl groups in one molecule, and can preferably have 2 to 5 (meth)acryloyl groups in one molecule, more preferably have 2 to 3 (meth)acryloyl groups in one molecule, further preferably have 2 (meth)acryloyl groups in one molecule.

The component (B) in the photocurable composition of the present invention preferably has no hydroxyl group in its molecule, from the viewpoint of a reduction in viscosity. The component (B) is preferably one having a -OR²- group (wherein R² is a divalent alkylene group) in its molecule. In one embodiment, the component (B) is a (meth)acrylate having an alkylene oxide-modified bisphenol backbone (another name: alkoxylated bisphenol backbone (meth)acrylate). For example, the component (B) is most preferably a compound represented by the following general formula 1.

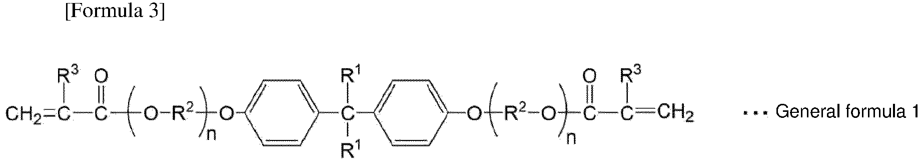

In the general formula 1, each R¹ independently represents a hydrogen atom or a monovalent alkyl group, each R² independently represents a divalent alkylene group, each R³ independently represents a hydrogen atom or methyl group, and n represents an integer of 1 or more.

The monovalent alkyl group is preferably a straight, branched or alicyclic alkyl group having 1 to 20 carbon atoms, more preferably a straight or branched alkyl group having 1 to 10 carbon atoms, further preferably a straight or branched alkyl group having 1 to 5 carbon atoms. Examples of the monovalent alkyl group include a methyl group, an ethyl group, a propyl group, a n-butyl group, a tert-butyl group, a n-hexyl group, and a n-octyl group. The divalent alkylene group is preferably a straight, branched or alicyclic alkylene group having 1 to 20 carbon atoms, more preferably a straight or branched alkylene group having 1 to 10 carbon atoms, further preferably a straight or branched alkylene group having 1 to 5 carbon atoms. Examples of the divalent alkylene group include a methylene group, an ethylene group, a propylene group, a n-butylene group, a n-hexylene group, and a n-octylene group.

Preferably, R² is a methyl group, R² is an ethylene group or a propylene group, R³ is a methyl group, and n is 1 or more and 5 or less.

In one embodiment, the component (B) is a (meth)acrylate monomer having a bisphenol backbone having 2 to 5 (preferably 2 to 3) (meth)acryloyl groups per molecule. In one embodiment, the component (B) is a (meth)acrylate monomer in which a (meth)acryloyl group is added to each of both ends of an alkylene oxide-modified bisphenol. Such a configuration can allow for rapid curing. Two or more such components (B) can also be used in combination.

The viscosity (25°C) of the component (B) is preferably 400 to 3000 mPa·s, more preferably 500 to 2500 mPa·s, further preferably 800 to 2000 mPa·s.

Specific examples of the component (B) include NK Ester BPE-80N, BPE-100, BPE-200, BPE-500, BPE-900, and BPE-1300N manufactured by Shin-Nakamura Chemical Co., Ltd.; and FA-324MG, FA-321M, and FA-3218M manufactured by Hitachi Chemical Co., Ltd., but not limited thereto.

The content of the component (B) in the photocurable composition of the present invention is preferably 10 parts by mass or more and 100 parts by mass or less, more preferably 20 parts by mass or more and 95 parts by mass or less, further preferably 30 parts by mass or more and 90 parts by mass or less, particularly preferably 40 parts by mass or more and 85 parts by mass or less, most preferably 50 parts by mass or more and 85 parts by mass or less based on 100 parts by mass of the component (A). A content of the component (B) of 10 parts by mass or more allows for formation of a cured product which is favorable in curability and which is hardly scratched, and a content of the component (B) of 100 parts by mass or less allows the composition to be lower in viscosity and easier in procedures. In a case where such two or more components (B) are used, the total content thereof is the content of the component (B).

### <Component (C)>

The photocurable composition of the present invention comprises, as the component (C), one or more selected from the group consisting of a component (c-1): a (meth)acrylate having an alicyclic hydrocarbon backbone: and a component (c-2): a (meth)acrylate having an isocyanurate backbone. One or more selected from the group consisting of a component (c-1): a (meth)acrylate having an alicyclic hydrocarbon backbone: and a component (c-2): a (meth)acrylate having an isocyanurate backbone are contained as the component (C) usable in the present invention. Herein, the component (C) is a component other than the component (A) and the component (B). The alicyclic hydrocarbon backbone represents a cycloaliphatic group and is saturated or unsaturated, and is here preferably a saturated cycloaliphatic group in order to maintain transparency. Herein, a (meth)acrylate having an alicyclic hydrocarbon backbone and an isocyanurate group is defined as the component (c-2). In other words, the component (c-1) does not include a (meth)acrylate having an isocyanurate group.

### · Component (c-1)

The component (c-1) may be a (meth)acrylate having an alicyclic hydrocarbon backbone, preferably a (meth)acrylate monomer having an alicyclic hydrocarbon backbone (cycloaliphatic group), more preferably one in which 2 or more (meth)acryloyl groups are bound to an alicyclic hydrocarbon backbone (cycloaliphatic group) directly or via a divalent linking group. The alicyclic hydrocarbon backbone is also referred to as "cycloaliphatic group". In a case where the component (C) comprises the component (c-1), it is necessary that no monofunctional (meth)acrylate be contained as a component (D) or the content of a monofunctional (meth)acrylate contained as a component (D) be 50% by mass or less based on the total mass of a component (D) described below. In a case where the component (C) contains the component (c-1) and also the content of a monofunctional (meth)acrylate contained as a component (D) is more than 50% by mass, wear resistance is decreased and the surface gloss after wiping off is decreased.

The component (c-1) preferably has 2 to 5, more preferably 2 to 3, further preferably 2 (meth)acryloyl groups in one molecule.

The alicyclic hydrocarbon backbone (cycloaliphatic group) in the component (c-1) may have an aliphatic branched chain, or may be an aliphatic group of a monocyclic ring or a polycyclic (dicyclic, tricyclic, or the like) ring serving as crosslinked ring. The cycloaliphatic group constituting the alicyclic hydrocarbon backbone is preferably a saturated or unsaturated cycloaliphatic group having 4 to 20 carbon atoms, more preferably a saturated or unsaturated cycloaliphatic group having 5 to 12 carbon atoms, further preferably a saturated or unsaturated cycloaliphatic group having 5 to 10 carbon atoms.

Examples of the cycloaliphatic group include a cyclopropane ring, a cyclobutane ring, a cyclopentane ring, a cyclopentene ring, a cyclohexane ring, a cyclohexene ring, a cycloheptane ring, a cyclooctane ring, a norbornane ring, a norbornene ring, a bornane ring, an adamantane ring, a tetrahydronaphthalene ring, a bicyclo[2.2.2]octane ring, a dicyclopentane ring, a dicyclopentadiene ring, a tricyclodecane ring, and a tricyclodecene ring. Accordingly, the component (c-1) is preferably a (meth)acrylate monomer having one or more cycloaliphatic groups selected from the group consisting of a cyclopropane ring, a cyclobutane ring, a cyclopentane ring, a cyclopentene ring, a cyclohexane ring, a cyclohexene ring, a cycloheptane ring, a cyclooctane ring, a norbornane ring, a norbornene ring, a bornane ring, an adamantane ring, a tetrahydronaphthalene ring, bicyclo[2.2.2]octane ring, a dicyclopentane ring, a dicyclopentadiene ring, a tricyclodecane ring and a tricyclodecene ring, as well as 2 to 5 (meth)acryloyl groups.

Examples of the component (c-1) include cyclohexyl (meth)acrylate, dicyclopentanyl (meth)acrylate, isobornyl (meth)acrylate, tricyclodecane dimethanol di(meth)acrylate, neopentyl glycol di(meth)acrylate, 1,6-hexane glycol di(meth)acrylate, dimethylol tricyclodecane di(meth)acrylate (another name: tricyclodecane dimethanol diacrylate), dicyclopentenyl di(meth)acrylate, and ethylene oxide-modified dicyclopentenyl di(meth)acrylate, but not limited thereto. It is particularly preferably to comprise dimethylol tricyclodecane diacrylate as the component (c-1). Two or more such components (c-1) can also be used in combination.

Specific examples of the component (c-1) include Light Acrylate series, such as DCP-A manufactured by Kyoeisha Chemical Co., Ltd., and IRR 214-K (EBECRYL 130) manufactured by Daicel-Allnex Ltd., but not limited thereto.

The content of the component (c-1) in the photocurable composition of the present invention is preferably 0.1 parts by mass or more and 100 parts by mass or less, more preferably 0.2 parts by mass or more and 80 parts by mass or less, further preferably 0.3 parts by mass or more and 70 parts by mass or less, particularly preferably 0.5 parts by mass or more and 60 parts by mass or less, most preferably 0.7 parts by mass or more and 55 parts by mass or less based on 100 parts by mass of the component (A). A content of the component (c-1) of 0.1 parts by mass or more results in an improvement in surface gloss after wiping off, and a content of the component (c-1) of 100 parts by mass or less results in an enhancement in coatability and an enhancement in workability. In a case where such two or more components (c-1) are used, the total content thereof is the content of the component (c-1).

### · Component (c-2)

The component (c-2) may be a (meth)acrylate having an isocyanurate backbone, and is preferably a (meth)acrylate monomer having an isocyanurate backbone, more preferably one in which 2 or more (meth)acryloyl groups are bound to an isocyanur isocyanurate backbone ate backbone directly or via a divalent linking group. The (meth)acrylate group having an isocyanurate backbone also corresponds to the component (c-2) when it has an alicyclic hydrocarbon backbone.

The component (c-2) preferably has 2 to 5, more preferably 2 to 3, further preferably 3 (meth)acryloyl groups in one molecule.

The component (c-2) is a compound having a structure represented by the following general formula 2.

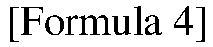

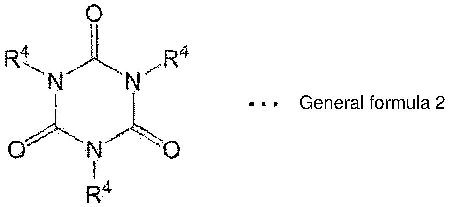

Here, each R⁴ is an independent organic group, and at least two organic groups in R⁴ each contain a (meth)acryloyl group.

Examples of the component (c-2) include tris(acryloyloxyethyl)isocyanurate, bis(acryloyloxyethyl)isocyanurate, and caprolactone-modified isocyanurate acrylate, but not limited thereto. The component (C) is particularly preferably a compound having 2 or more (meth)acryloyl groups in one molecule from the viewpoint of expression of gloss on a surface after curing. The component (C) particularly preferably comprises, as the component (c-2), caprolactone-modified tris-(2-acryloxyethyl)isocyanurate. Two or more such components (c-2) can also be used in combination.

The content of the component (c-2) in the photocurable composition of the present invention is preferably 0.1 parts by mass or more and 100 parts by mass or less, more preferably 0.5 parts by mass or more and 80 parts by mass or less, further preferably 1 part by mass or more and 70 parts by mass or less, particularly preferably 5 parts by mass or more and 60 parts by mass or less, most preferably 10 parts by mass or more and 50 parts by mass or less based on 100 parts by mass of the component (A). A content of the component (c-2) of 0.1 parts by mass or more results in an improvement in surface gloss after wiping off, and a content of the component (c-2) of 100 parts by mass or less results in an enhancement in coatability and an enhancement in workability. In a case where two or more such components (c-2) are used, the total content thereof is the content of the component (c-2).

Specific examples of the component (c-2) include NK Ester A9300, A9300-1CL, and A-9200YN manufactured by Shin-Nakamura Chemical Co., Ltd., but not limited thereto.

The amount of the component (C) contained is preferably 0.1 to 100 parts by mass, more preferably 0.1 to 80 parts by mass, further preferably 0.1 to 60 parts by mass, particularly preferably 0.5 to 55 parts by mass based on 100 parts by mass of the component (A). When the amount of the component (C) contained is 0.1 parts by mass or more, the composition is lowered in viscosity and easier in procedures, and when the amount of the component (C) contained is 100 parts by mass or less, curability is favorable. Although no clear reason is found, in a case where the component (C) comprises the component (c-1), it is necessary for expression of gloss after curing that no monofunctional (meth)acrylate be contained as a component (D) described below or the content of a monofunctional (meth)acrylate contained as a component (D) be 50% by mass or less based on the total mass of a component (D). Both the component (c-1) and the component (c-2) have a cyclic backbone, and it is presumed that a certain or more amount of a monofunctional (meth)acrylate leading to a reduction in crosslinking density is added to result in the difference in gloss of a cured product. In a case where the component (c-1) and the component (c-2) are used, the total content thereof is the content of the component (C).

### <Component (D)>

The photocurable composition of the present invention comprises, as the component (D), any (meth)acrylate other than the components (A) to (C). In other words, the component (D) is any (meth)acrylate excluding the urethane-modified (meth)acrylate oligomer and the (meth)acrylates respectively having a bisphenol backbone, an alicyclic hydrocarbon backbone and an isocyanurate backbone. The component (D) can be used singly or in combinations of two or more kinds thereof.

The component (D) usable in the present invention is a (meth)acrylate having one or more (meth)acryloyl groups per molecule (herein, the component (D) is other than the components (A) to (C)). Any compound having a (meth)acryloyl group, other than the components (A) to (C), can be used as the component (D). Specifically, a (meth)acrylate monomer or a (meth)acrylate oligomer is included as the component (D). Herein, the "oligomer" refers to a compound having a molecular weight or weight average molecular weight of more than 1000, among compounds each having a structure in which a plurality of repeating units (monomer units) is bound, and such a compound itself, even if further formed into a multimer by polymerization or the like, is not referred to as "monomer", but referred to as "oligomer", unless otherwise described. Herein, a compound having a molecular weight or weight average molecular weight of 1000 or less, among compounds each having a structure in which a plurality of repeating units (monomer units) is bound, is referred to as "monomer". The component (D) is preferably in the form of a liquid under an atmosphere at 25°C, and can be suitably used as long as it has favorable compatibility with the components (A) to (C) in the present invention.

The component (D) is preferably any (meth)acrylate monomer other than the components (B) to (C), and is any (meth)acrylate monomer excluding the (meth)acrylates respectively having a bisphenol backbone, an alicyclic hydrocarbon backbone and an isocyanurate backbone. The component (D) preferably has 1 to 6 (meth)acryloyl groups per molecule, more preferably has 1 to 4 (meth)acryloyl groups per molecule, further preferably has 1 to 3 (meth)acryloyl groups per molecule, in consideration of heat generation during photocuring. The component (D) is preferably one or more selected from the group consisting of a monofunctional (meth)acrylate, a difunctional (meth)acrylate, and a trifunctional (meth)acrylate.

Specific examples of the (meth)acrylate oligomer include a (meth)acrylate oligomer having an ester bond in its molecule, but not limited thereto. The component (A) usable in the present invention also encompasses a compound having one or more epoxy groups and one or more (meth)acryloyl groups in one molecule.

A known method for synthesizing the (meth)acrylate oligomer having an ester bond is a synthesis method including forming an ester bond by a polyol and a polyvalent carboxylic acid and adding acrylic acid to an unreacted hydroxyl group, but not limited to this synthesis method. Specific examples thereof include Aronix M-6100, M-6200, M-6250, M-6500, M-7100, M-7300K, M-8030, M-8060, M-8100, M-8530, M-8560, and M-9050 manufactured by Toagosei Co., Ltd., and UV-3500BA, UV-3520TL, UV-3200B, and UV-3000B manufactured by Nippon Synthetic Chemical Industry Co., Ltd., but not limited thereto.

The (meth)acrylate monomer can include mono- to hexafunctional (preferably monofunctional, difunctional or trifunctional) (meth)acrylate monomer and (meth)acrylamide monomer. A plurality of any other monomers can also be used in combination.

Specific examples of the monofunctional (meth)acrylate monomer include lauryl (meth)acrylate, stearyl (meth)acrylate, ethyl carbitol (meth)acrylate, tetrahydrofurfuryl (meth)acrylate, caprolactone-modified tetrahydrofurfuryl (meth)acrylate, benzyl (meth)acrylate, phenyl (meth)acrylate, phenoxyethyl (meth)acrylate, phenoxydiethylene glycol (meth)acrylate, phenoxytetraethylene glycol (meth)acrylate, nonylphenoxyethyl (meth)acrylate, nonylphenoxytetraethylene glycol (meth)acrylate, methoxydiethylene glycol (meth)acrylate, ethoxydiethylene glycol (meth)acrylate, butoxyethyl (meth)acrylate, butoxytriethylene glycol (meth)acrylate, 2-ethylhexylpolyethylene glycol (meth)acrylate, 4-hydroxybutyl (meth)acrylate, nonylphenylpolypropylene glycol (meth)acrylate, methoxydipropylene glycol (meth)acrylate, 2-hydroxyethyl (meth)acrylate, 2-hydroxypropyl (meth)acrylate, glycerol (meth)acrylate, polyethylene glycol (meth)acrylate, polypropylene glycol (meth)acrylate, N,N-dimethylaminoethyl (meth)acrylate, and N,N-diethylaminoethyl (meth)acrylate, but not limited thereto. The component (D) is preferably a (meth)acrylate monomer having a hydroxyl group, and is, for example, preferably 4-hydroxybutyl (meth)acrylate, 2-hydroxyethyl (meth)acrylate, or 2-hydroxypropyl (meth)acrylate.

Examples of the monofunctional (meth)acrylate monomer also include a (meth)acrylate monomer having an acidic group. The (meth)acrylate monomer having an acidic group particularly refers to carboxylic acid, phosphoric acid, or the like having a (meth)acryloyl group in its molecule. Examples of the carboxylic acid having a (meth)acryloyl group in its molecule include (meth)acryloyl acid, 3-(meth)acryloyloxypropylsuccinic acid, 4-(meth)acryloyloxybutylsuccinic acid, 2-(meth)acryloyloxyethylmaleic acid, 3-(meth)acryloyloxypropylmaleic acid, 4-(meth)acryloyloxybutylmaleic acid, 2-(meth)acryloyloxyethylhexahydrophthalic acid, 3-(meth)acryloyloxypropylhexahydrophthalic acid, 4-(meth)acryloyloxybutylhexahydrophthalic acid, 2-(meth)acryloyloxyethylphthalic acid, 3-(meth)acryloyloxypropylphthalic acid, and 4-(meth)acryloyloxybutylphthalic acid, and examples of the phosphoric acid having a (meth)acryloyl group in its molecule include 2-ethylhexyl acid phosphate, 2-hydroxyethyl methacrylate acid phosphate, and dibutyl phosphate, but not limited thereto.

Specific examples of the difunctional (meth)acrylate monomer include 1,3-butylene glycol di(meth)acrylate, 1,4-butylene glycol di(meth)acrylate, ethylene glycol diacrylate, tetraethylene glycol di(meth)acrylate, polyethylene glycol di(meth)acrylate, propylene glycol di(meth)acrylate, tripropylene glycol di(meth)acrylate, ethylene oxide-modified neopentyl glycol di(meth)acrylate, propylene oxide-modified neopentyl glycol di(meth)acrylate, and neopentyl glycol-modified trimethylolpropane di(meth)acrylate, but not limited thereto.

Specific examples of the trifunctional (meth)acrylate monomer include trimethylolpropane tri(meth)acrylate, trimethylolpropane tri(meth)acrylate, pentaerythritol tri(meth)acrylate, ethylene oxide-modified trimethylolpropane tri(meth)acrylate (another name: ethoxylated trimethylolpropane tri(meth)acrylate), and propylene oxide-modified trimethylolpropane tri(meth)acrylate, but not limited thereto.

Specific examples of the polyfunctional (meth)acrylate monomer which is tetra- or higher functional group include ditrimethylolpropane tetra(meth)acrylate, pentaerythritol tetra(meth)acrylate, dipentaerythritol monohydroxy penta(meth)acrylate, alkyl-modified dipentaerythritol penta(meth)acrylate, and dipentaerythritol hexa(meth)acrylate.

The component (D) preferably contains, as a monofunctional (meth)acrylate, a monofunctional (meth)acrylate having a hydroxyl group in its molecule from the viewpoint of expression of surface curability and gloss.

The component (D) is preferably one or more selected from the group consisting of a monofunctional (meth)acrylate, a difunctional (meth)acrylate and a trifunctional (meth)acrylate. The component (D) preferably does not contain a tetra- or higher functional (meth)acrylate from the viewpoint of suppression of heat generation during procedures.

The component (D) preferably contains one or more selected from the group consisting of a (meth)acrylate monomer having a hydroxyl group and 1 or 2 (meth)acryloyl groups; and a (meth)acrylate monomer having 1 to 3 (meth)acryloyl groups; more preferably contains one or more selected from the group consisting of a (meth)acrylate monomer having a hydroxyl group and 1 or 2 (meth)acryloyl groups; and a (meth)acrylate monomer having 2 or 3 (meth)acryloyl groups; further preferably contains one or more selected from the group consisting of a (meth)acrylate monomer having a hydroxyl group and one (meth)acryloyl group; and a (meth)acrylate monomer having 3 (meth)acryloyl groups; particularly preferably contains a (meth)acrylate monomer having a hydroxyl group and one (meth)acryloyl group; and a (meth)acrylate monomer having 3 (meth)acryloyl groups.

The component (D) in the photocurable composition of the present invention preferably has a -OR⁶-group (wherein R⁶ is a divalent alkylene group) in its molecule from the viewpoint from compatibility with the component (B). Examples of the alkylene group as R⁶ include an ethylene group and a propylene group. In one embodiment, the component (D) is an alkylene oxide-modified (meth)acrylate monomer.

The content of the component (D) is preferably 1 part by mass or more and 60 parts by mass or less, more preferably 2 parts by mass or more and 55 parts by mass or less, further preferably 5 parts by mass or more and 50 parts by mass or less, particularly preferably 8 parts by mass or more and 40 parts by mass or less, most preferably 10 parts by mass or more and 30 parts by mass or less based on 100 parts by mass of the component (A). A content of the component (D) of 1 part by mass or more allows the composition to be lower in viscosity and easier in procedures, and a content of the component (D) of 60 parts by mass or less results in favorable curability. In a case where two or more such components (D) are used, the total content thereof is the content of the component (D). In a case where the photocurable composition of the present invention contains the component (c-1), no monofunctional (meth)acrylate is contained as a component (D) described below or the content of a monofunctional (meth)acrylate contained as a component (D) is 50% by mass or less based on the total mass of a component (D) described below.

### <Component (E)>

The component (E) usable in the present invention is a photoinitiator. The component (E) is not limited as long as it is a radical photoinitiator which generates radical species by an energy ray such as visible light, ultraviolet light, X-ray, or electron beam.

Specific examples of the component (E) include acetophenones such as diethoxyacetophenone, 2-hydroxy-2-methyl-1-phenylpropan-1-one, benzyl dimethyl ketal, 4-(2-hydroxyethoxy)phenyl-(2-hydroxy-2-propyl)ketone, 1-hydroxycyclohexyl phenyl ketone, 2-methyl-2-morpholino(4-thiomethylphenyl)propan-1-one, 2-benzyl-2-dimethylamino-1-(4-morpholinophenyl)butanone, and a 2-hydroxy-2-methyl-1-[4-(1-methylvinyl)phenyl]propanone oligomer; benzoins such as benzoin, benzoin methyl ether, benzoin ethyl ether, benzoin isopropyl ether, and benzoin isobutyl ether; benzophenones such as benzophenone, methyl o-benzoylbenzoate, 4-phenylbenzophenone, 4-benzoyl-4'-methyl-diphenyl sulfide, 3,3',4,4'-tetra(t-butylperoxycarbonyl)benzophenone, 2,4,6-trimethylbenzophenone, 4-benzoyl-N,N-dimethyl-N-[2-(1-oxo-2-propenyloxy)ethyl]benzenemethanaminium bromide, and (4-benzoylbenzyl)trimethylammonium chloride; and thioxanthones such as 2-isopropylthioxanthone, 4-isopropylthioxanthone, 2,4-diethylthioxanthone, 2,4-dichlorothioxanthone, 1-chloro-4-propoxythioxanthone, and 2-(3-dimethylamino-2-hydroxy)-3,4-dimethyl-9H-thioxanthon-9-one mesochloride, but not limited thereto. A plurality of such components (E) can also be used in combinations thereof.

The content of the component (E) is preferably 0.1 parts by mass or more and 20 parts by mass or less, more preferably 1 part by mass or more and 18 parts by mass or less, further preferably 5 parts by mass or more and 5 parts by mass or less based on 100 parts by mass of the component (A). When the content of the component (E) is 0.1 parts by mass or more, photocurability can be maintained. On the other hand, when the content of the component (E) is 20 parts by mass or less, storage stability can be maintained without thickening during storage. The amount of the visible light type photoinitiator contained is preferably 0% by mass or more and 70% by mass or less relative to the entire component (E), and yellowing of a cured product is hardly caused. The visible light type photoinitiator is here a photoinitiator which most strongly absorbs light in the visible region and which mainly represents an acyl phosphine oxide-based photopolymerization initiator containing a phosphorus atom. Specific examples of the visible light type photoinitiator include 2,4,6-trimethylbenzoyl-diphenyl-phosphine oxide and bis(2,4,6-trimethylbenzoyl)-phenylphosphine oxide, but not limited thereto.

### <Other component>

In the present invention, an ultraviolet absorber can be further included. Herein, the ultraviolet absorber is a component other than the components (A) to (E). A preferred ultraviolet absorber is a compound having one phenol group in its molecule, and is, for example, a phenol compound represented by the following general formula 3. Herein, only one among R⁵ is an organic group conjugated with a phenol aromatic ring, and the remaining R⁵ are each independently a hydrogen atom or a chain-type organic group, and the chain-type organic group contains neither an aromatic ring, nor an alicyclic structure. The molecular weight of the ultraviolet absorber is preferably 150 to 600 from the viewpoint of solubility in the component (A) and component (B). The ultraviolet absorber cab be added in order to suppress heat generation during curing.

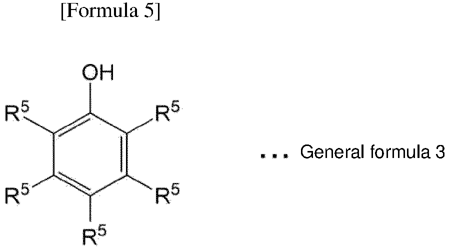

The organic group conjugated with a phenol aromatic ring in the phenol compound represented by the general formula 3 is preferably a group of the following formula 4 or a group of the following formula 5 or a group of the following formula 6, or a group having a backbone of the following formula 4, the following formula 5 or the following formula 6. Examples of such an ultraviolet absorber include 2-hydroxy-4-n-octyloxybenzophenone, but not limited thereto.

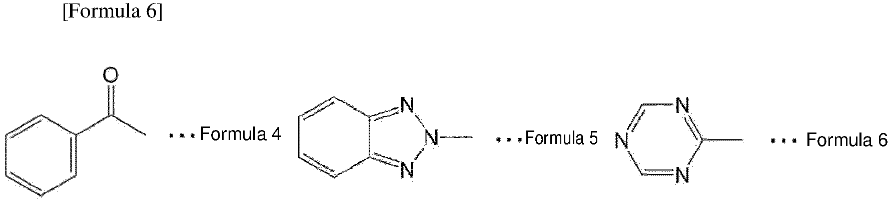

The content of the ultraviolet absorber is preferably 0.01 to 5.0 parts by mass based on 100 parts by mass of the component (A). When the content of the ultraviolet absorber is 0.01 parts by mass or more, heat generation during photocuring can be suppressed, and when the content is 5.0 parts by mass or less, photocurability, in particular, surface curability can be maintained. The amount of the ultraviolet absorber contained is preferably 0.001 to 10.0% by mass relative to the entire composition. When the amount of the ultraviolet absorber contained is 0.001% by mass or more, heat generation can be suppressed, and when the amount is 10.0% by mass or less, storage stability can be maintained well.

A proper amount of additive(s) such as a polythiol compound, a (meth)acrylamide monomer, a coupling agent, an inorganic filling agent or an organic filling agent, a colorant such as a pigment or a dye, an antioxidant, a polymerization inhibitor, a defoaming agent, a leveling agent, and/or a rheology control agent may be contained in the photocurable composition of the present invention, as long as features of the present invention are not impaired. Such addition provides a composition excellent in resin strength, workability, storage stability, and the like, or a cured product thereof.

A polythiol compound may be contained in the photocurable composition of the present invention, as long as features of the present invention are not impaired. The polythiol compound is a compound having two or more thiol groups in one molecule. While photocurable compositions to which polythiol compounds are added are known, such compositions have inherent odor and, when contacted with decorative objects of silver, are oxidized and thus progressively blackened. Specific examples of the polythiol compound include an aliphatic polythiol compound, an aromatic polythiol compound, and a polythiol compound having a sulfide bond, but not limited thereto.

Examples of an aliphatic polythiol compound having two thiol groups include 1,2-ethanedithiol, 1,2-propanedithiol, 1,3-propanedithiol, 1,4-butanedithiol, 1,6-hexanedithiol, 1,7-heptanedithiol, 1,8-octanedithiol, 1,9-nonanedithiol, 1,10-decanedithiol, 1,12-dodecanedithiol, 2,2-dimethyl-1,3-propanedithiol, 3-methyl-1,5-pentanedithiol, 2-methyl-1,8-octanedithiol, 1,4-cyclohexanedithiol, 1,4-bis(mercaptomethyl)cyclohexane, 1,1-cyclohexanedithiol, 1,2-cyclohexanedithiol, bicyclo[2,2,1]hepta-exo-cis-2,3-dithiol, 1,1-bis(mercaptomethyl)cyclohexane, bis(2-mercaptoethyl)ether, ethylene glycol bis(2-mercaptoacetate), and ethylene glycol bis(3-mercaptopropionate), but not limited thereto.

Examples of an aliphatic polythiol compound having three thiol groups include 1,1,1-tris(mercaptomethyl)ethane, 2-ethyl-2-mercaptomethyl-1,3-propanedithiol, 1,2,3-propanetrithiol, trimethylolpropane tris(2-mercaptoacetate), trimethylolpropane tris(3-mercaptopropionate), and tris[(mercaptopropynyloxy)-ethyl]isocyanurate, but not limited thereto.

Examples of an aliphatic polythiol compound having four or more thiol groups include pentaerythritol tetrakis(2-mercaptoacetate), pentaerythritol tetrakis(3-mercaptopropionate), pentaerythritol tetrakis(3-mercaptobutyrate), and dipentaerythritol hexa-3-mercaptopropionate, but not limited thereto.

Examples of the aromatic polythiol compound include 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(2-mercaptoethyl)benzene, 1,3-bis(2-mercaptoethyl)benzene, 1,4-bis(2-mercaptoethyl)benzene, 1,2-bis(2-mercaptoethyleneoxy)benzene, 1,3-bis(2-mercaptoethyleneoxy)benzene, 1,4-bis(2-mercaptoethyleneoxy)benzene, 1,2,3-trimercaptobenzene, 1,2,4-trimercaptobenzene, 1,3,5-trimercaptobenzene, 1,2,3-tris(mercaptomethyl)benzene, 1,2,4-tris(mercaptomethyl)benzene, 1,3,5-tris(mercaptomethyl)benzene, 1,2,3-tris(2-mercaptoethyl)benzene, 1,2,4-tris(2-mercaptoethyl)benzene, 1,3,5-tris(2-mercaptoethyl)benzene, 1,2,3-tris(2-mercaptoethyleneoxy)benzene, 1,2,4-tris(2-mercaptoethyleneoxy)benzene, 1,3,5-tris(2-mercaptoethyleneoxy)benzene, 1,2,3,4-tetramercaptobenzene, 1,2,3,5-tetramercaptobenzene, 1,2,4,5-tetramercaptobenzene, 1,2,3,4-tetrakis(mercaptomethyl)benzene, 1,2,3,5-tetrakis(mercaptomethyl)benzene, 1,2,4,5-tetrakis(mercaptomethyl)benzene, 1,2,3,4-tetrakis(2-mercaptoethyl)benzene, 1,2,3,5-tetrakis(2-mercaptoethyl)benzene, 1,2,4,5-tetrakis(2-mercaptoethyl)benzene, 1,2,3,4-tetrakis(2-mercaptoethyleneoxy)benzene, 1,2,3,5-tetrakis(2-mercaptoethyleneoxy)benzene, 1,2,4,5-tetrakis(2-mercaptoethyleneoxy)benzene, 2,2'-mercaptobiphenyl, 4,4'-thiobis-benzenethiol, 4,4'-dimercaptobiphenyl, 4,4'-dimercaptobibenzyl, 2,5-toluenedithiol, 3,4-toluenedithiol, 1,4-naphthalenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-bis(2-mercaptoethylthio)benzene, 1,4-bis(2-mercaptoethylthio)benzene, 1,2-bis(2-mercaptoethylthiomethyl)benzene, 1,3-bis(2-mercaptoethylthiomethyl)benzene, 1,4-bis(2-mercaptoethylthiomethyl)benzene, 1,2,3-tris(2-mercaptoethylthio)benzene, 1,2,4-tris(2-mercaptoethylthio)benzene, 1,3,5-tris(2-mercaptoethylthio)benzene, 1,2,3,4-tetrakis(2-mercaptoethylthio)benzene, 1,2,3,5-tetrakis(2-mercaptoethylthio)benzene, and 1,2,4,5-tetrakis(2-mercaptoethylthio)benzene, but not limited thereto.

Examples of the polythiol compound having a sulfide bond include bis(2-mercaptoethyl)sulfide, bis(2-mercaptoethylthio)methane, 1,2-bis(2-mercaptoethylthio)ethane, 1,3-bis(2-mercaptoethylthio)propane, 1,2,3-tris(2-mercaptoethylthio)propane, tetrakis(2-mercaptoethylthiomethyl)methane, 1,2-bis(2-mercaptoethylthio)propanethiol, 2,5-dimercapto-1,4-dithiane, bis(2-mercaptoethyl)disulfide, 3,4-thiophenedithiol, 1,2-bis(2-mercaptoethyl)thio-3-mercaptopropane, and bis-(2-mercaptoethylthio-3-mercaptopropane)sulfide, but not limited thereto.

Specific examples of the polythiol compound having a secondary thiol group include pentaerythritol tetrakis(3-mercaptobutyrate), 1,4-bis(3-mercaptobutyryloxy)butane, 1,3,5-tris(3-mercaptobutyryloxyethyl)-1,3,5-triazin-2,4,6(1H,3H,5H)-trione, trimethylolpropane tris(3-mercaptobutyrate), trimethylolethane tris(3-mercaptobutyrate), trimethylolpropane tris(3-mercaptobutyrate), and trimethylolethane tris(3-mercaptobutyrate), but not limited thereto. Examples of a commercial product include PEMP manufactured by SC Organic Chemical Co., Ltd., and KarenzMT (registered trademark) series, PE1, BD1, and NR1 manufactured by Showa Denko K.K., but not limited thereto.

A (meth)acrylamide monomer can be added to the photocurable composition of the present invention as long as features of the present invention are not impaired. Specific examples include dimethyl(meth)acrylamide, (meth)acryloylmorpholine, and diethyl(meth)acrylamide, but not limited thereto. Although no clear reason is found, the monomer preferably contains the (meth)acrylamide monomer from the viewpoint of an enhancement in durability. DMAA, ACMO, DEAA, and the like manufactured by KJ Chemicals Corporation are known as specific examples of the (meth)acrylamide monomer in the present invention, without limitation thereto.

In the present invention, a coupling agent can be added as long as features of the present invention are not impaired. Examples of the coupling agent include a silane-based coupling agent having an epoxy group, a vinyl group, an acryloyl group or a methacryloyl group, and also a hydrolyzable silane group in combination, a polyorganosiloxane having a phenyl group and a hydrolyzable silyl group, and/or a polyorganosiloxane having an epoxy group and a hydrolyzable silyl group, but not limited thereto. Specific examples of the silane-based coupling agent include allyltrimethoxysilane, vinyltrimethoxysilane, 3-glycidoxypropyltrimethoxysilane, 3-methacryloxypropyltrimethoxysilane, 3-acryloxypropyltrimethoxysilane, and 3-chloropropyltrimethoxysilane, but not limited thereto.

A filling agent such as an inorganic filling agent and an organic filling agent can be added in the photocurable composition of the present invention, as long as features of the present invention are not impaired. Such a filling agent can be added to adjust not only viscous properties/thixotropy, but also curability and toughness. Examples of the inorganic filling agent include alumina, silica, and amorphous silica, but not limited thereto. On the other hand, examples of the organic filling agent include a styrene filler, a rubber filler, and a core-shell acrylic filler, but not limited thereto. Examples of a specific product of silica include FUSELEX E-1 manufactured by Tatsumori Ltd. and AO-802 manufactured by Admafine, and examples of a specific product of amorphous silica include Aerosil series, 200 (not treated), R972 (treated with dimethyldichlorosilane), R976 (treated with dimethyldichlorosilane), RY200 (treated with dimethylsilicone), RX200 (treated with hexamethyldisilazane), and R800 (treated with octylsilane) manufactured by Nippon Aerosil Co., Ltd., but not limited thereto.

The method for preparing the photocurable composition for a nail of the present invention is not particularly limited, and a conventionally known method can be appropriately adopted. For example, the component (A), the component (B), the component (C), the component (D), the component (E), and a component optionally added are respectively weighed in predetermined amounts, added to a stirring oven sequentially in any order or simultaneously, and then mixed by use of a mixing unit such as a planetary mixer preferably with defoaming in vacuum. A preferred order of addition is set so that the component (E) is added at the end. The component (E) can be added at the end to prevent photopolymerization reaction from progressing at an undesired stage. Here, production conditions are not particularly limited, and production is preferably performed under light shielding conditions. The mixing temperature is preferably a temperature of 10 to 50°C, and the mixing time is preferably 0.1 to 5 hours.

When the photocurable composition for a nail or an artificial nail of the present invention is used in a so-called gel nail, procedures are performed as follows. Before such procedures, sanding of the surface of a human nail by a file or the like is performed and then any dust, oil, water, and the like are removed by a nail-specific solvent mainly containing ethanol. Preferably, in the case of coating with the photocurable composition of the present invention, a coating film having a thickness of 100 to 300 µm before curing is formed by a pencil or a brush. A primer may also be used in advance in the coating. A commercially available LED lamp for gel nails is used as the irradiation apparatus in curing. The irradiation time is 15 seconds to 120 seconds, and is preferably 20 to 70 seconds in consideration of the influence on a finger.

The viscosity at 25°C of the photocurable composition for a nail or an artificial nail of the present invention is preferably 10 Pa·s or less, more preferably 5 Pa·s or less in consideration of coatability during procedures.

It is known that polymerization is suppressed by oxygen inhibition in a region where a compound having a (meth)acryloyl group is in touch with oxygen when polymerized with generation of radical species by an energy ray. An uncured component here remains on a cured product surface, thus such an uncured component is often wiped off by a waste cloth, cotton, or the like, or wiped off by a waste cloth or the like impregnated with a solvent such as ethanol, during procedures, and such wiping off is more likely performed particularly with respect to a topcoat used for the outermost surface. The present invention not only causes no scratch on a cured product surface even with wiping off performed by a waste cloth or the like, but also allows gloss to be expressed, and thus is suitable for a topcoat.

The initial glossiness (glossiness after curing and before a wear test) of a cured product obtained by photocuring the photocurable composition for a nail or an artificial nail according to the present invention is preferably 85% or more, more preferably 87% or more, further preferably 88% or more. The glossiness after a wear test of a cured product obtained by photocuring the photocurable composition for a nail or an artificial nail according to the present invention is preferably 83% or more, more preferably 85% or more, further preferably 88% or more. The change in glossiness before and after the wear test is here preferably 5.0% or less, more preferably 4.0% or less, further preferably 3.0% or less, particularly preferably 2.0% or less. Herein, measurement of the glossiness can be achieved by a method described in Examples. Herein, the wear test is performed by a method described in Examples.

In the present invention, while the numerical value obtained by measurement of the glossiness with a high gloss checker manufactured by Horiba Ltd., serves as one for reference purposes of the degree of gloss of a cured product, the presence or absence of luster by visual evaluation of the entire surface of such a cured product is regarded as being definitely evaluated as an actual degree of gloss. Accordingly, whether the result of visual evaluation of surface gloss is Good or Poor is preferentially considered as gloss evaluation. For example, when the gloss of a plurality of cured products evaluated as Good is compared, the glossiness calculated with the high gloss checker can serve as an index, to compare the degree of gloss, and when the gloss of a plurality of cured products evaluated as Poor is again compared, the glossiness calculated with the high gloss checker can serve as an index, to compare the degree of gloss.

### Example

Next, the present invention is further specifically described with reference to Examples, but the present invention is not limited only to these Examples.

### [Examples 1 to 7 and Comparative Examples 1 to 3]

The following components were provided for preparing a photocurable composition (hereinafter, the photocurable composition for a nail or an artificial nail being also simply referred to as "composition".).

### <<Component (A): urethane-modified (meth)acrylate oligomer>>

· Polyether urethane acrylate having a weight average molecular weight of 3500 and the number of functional groups of 2 (Art Resin UN-6303 manufactured by Negami Chemical Industrial Co., Ltd.)

### <<Component (B): (meth)acrylate having bisphenol backbone>>

· Ethoxylated bisphenol A dimethacrylate (NKester BPE-80N manufactured by Shin-Nakamura Chemical Co., Ltd.) (viscosity (25°C): 1220 mPa·s)

### <<Component (C): (meth)acrylate(s) of component (c-1) and/or component (c-2) (except for component (A) and component (B))>>

Component (c-1): (meth)acrylate having alicyclic hydrocarbon backbone
   · Dimethylol tricyclodecane diacrylate (Light Acrylate DCP-A manufactured by Kyoeisha Chemical Co., Ltd.)
Component (c-2): (meth)acrylate having isocyanurate backbone
   · Caprolactone-modified tris-(2-acryloxyethyl)isocyanurate (NK EsterA9300-1CL manufactured by Shin-Nakamura Chemical Co., Ltd.)

### <<Component (D): (meth)acrylate other than components (A) to (C)>>

· Ethoxylated (9) trimethylolpropane triacrylate (Sartomer SR502 manufactured by Arkema)
· Dipentaerythritol hexaacrylate (DPHA manufactured by Daicel-Allnex Ltd.)
· 4-Hydroxybutyl acrylate (4-HBA manufactured by Osaka Organic Chemical Industry Ltd.))

### <<Component (E): photoinitiator>>

· 1-Hydroxycyclohexyl phenyl ketone (non-visible light type photoinitiator) (IRGACURE 184, manufactured by BASF SE)
· 2,4,6-Trimethylbenzoyl-diphenyl-phosphine oxide (visible light type photoinitiator) (LUCIRIN TPO manufactured by BASF SE)

### <<Ultraviolet absorber>>

· 2-Hydroxy-4-n-octyloxybenzophenone (molecular weight: 326) (reagent) (hereinafter, abbreviated as octabenzone.)

In Examples 1 to 7 and Comparative Examples 1 to 3, compounding in amounts in Table 1 below was performed for preparation. Each composition was added by weighing the components (A) to (D) and the ultraviolet absorber to a stirring oven and then stirring them for 30 minutes, and thereafter weighing the component (E) and adding therein, and then stirring them for 30 minutes with defoaming in vacuum. The detailed amounts of preparation are as shown in Table 1, and all numerical values are expressed by "part(s) by mass". The total of the component (B), the total of the component (C), the total of the component (D), and the proportion (% by mass) of the monofunctional monomer relative to the total of the component (D) is also described.

**[Table 1]**

| Component | Raw material | Example 1 | Example 2 | Example 3 | Example 4 | Example *5* | Example 6 | Example 7 | Comparat ive Example 1 | Comparat ive Example 2 | Comparat ive Example 3 |
|---|---|---|---|---|---|---|---|---|---|---|---|
| Component (A) | UN-6303 | 100 | 100 | 100 | 100 | 100 | 100 | 100 | 100 | 100 | 100 |
| Component (B) | BPE-80N | 83 | 60 | 60 | 60 | 21 | 60 | 60 | | 60 | 60 |
| Component (c-1) | DCP-A | 1 | 14 | 14 | 14 | 53 | | | 74 | 27 | 3 |
| Component (c-2) | A9300-1CL | | | | | | 27 | 27 | | | |
| Component (D) | SR502 | 13.5 | 13.5 | 13.5 | | 13.5 | | | 13.5 | | 13.5 |
| | DPHA | | | | 13.5 | | | | | | |
| | 4-HBA | | 10.4 | 10.4 | 10.4 | 10.4 | 10.4 | 10.4 | 10.4 | 10.4 | 20.8 |
| Component (E) | 184 | 5.2 | 5.2 | 5.2 | 5.2 | 5.2 | 5.2 | 5.2 | 5.2 | 5.2 | 5.2 |
| | TPO | 5.2 | 5.2 | 5.2 | 5.2 | 5.2 | 5.2 | 5.2 | 5.2 | 5.2 | 5.2 |
| Ultraviolet absorber | Octabenzone | 1.0 | 1.0 | | 1.0 | 1.0 | 1.0 | | 1.0 | 1.0 | 1.0 |
| Total (parts by mass) | | 208.9 | 209.3 | 208.3 | 209.3 | 209.3 | 208.7 | 207.7 | 209.3 | 208.8 | 208.7 |
| Total of component (B) (parts by mass) | | 83 | 60 | 60 | 60 | 21 | 60 | 60 | 0 | 60 | 60 |
| Total of component (C) (parts by mass) | | 1 | 14 | 14 | 14 | 53 | 27 | 27 | 74 | 27 | 3 |
| Total of component (D) (parts by mass) | | 14 | 24 | 24 | 24 | 24 | 10 | 10 | 24 | 10 | 34 |
| Proportion of monofunctional monomer based on total of component (D) (% by mass) | | 0 | 44 | 44 | 44 | 44 | 100 | 100 | 44 | 100 | 61 |

In Examples 1 to 7 and Comparative Examples 1 to 3, evaluation of the cured product surface state (before wiping off), evaluation of the cured product surface state (after wiping off), measurement of the glossiness (before wiping off), measurement of the glossiness (after wiping off), evaluation of heat generation during procedures, and measurement of the degree of yellowness were performed. The results are summarized in Table 2.

### [Cured product surface state (before wiping off): "surface gloss (before wiping off)"]

A black coating fake nail was coated with the composition at a thickness of 100 µm, and the composition was cured by a LED lamp for nails (rated voltage: 100 to 110 V, consumed power at 50 to 60 Hz: 36 W, wavelength: 350 to 400 nm) for 30 seconds, to produce a test piece. The test piece is subjected to reflection onto a LED stand, and visually confirmed according to the following evaluation criteria, and "surface gloss (before wiping off)" is determined. "Good" is preferred.

### <<Evaluation criteria>>

Good: luster on surface
Poor: no luster on surface.

### [Evaluation of cured product surface state (after wiping off): "surface gloss (after wiping off)"]

The surface of the test piece whose surface was confirmed by the above cured product surface state test (before wiping off) was wiped five times by a waste cloth plentifully impregnated with acetone, and again subjected to reflection onto a LED stand and visually confirmed according to the following evaluation criteria, and "surface gloss (after wiping off)" was determined. "Good" is preferred.

### <<Evaluation criteria>>

Good: luster on surface
Poor: surface finely scratched.

### [Measurement of glossiness (before wiping off): "glossiness (%) (before wiping off)"]

A test piece (size: 0.8 mm thickness × 70 mm width × 150 mm length) where one surface of SPCC-SD as a substrate was subjected to electrodeposition coating with clear amino alkyd paint was used, the surface subjected to electrodeposition coating was coated with the composition at a thickness of 100 µm, and the composition was cured by irradiation with light with a wavelength of 365 nm at a cumulative amount of light of 30 kJ/m² by a belt conveyor type ultraviolet irradiator where high-pressure mercury lamp was installed, to produce a test piece. The glossiness of a cured product surface of the test piece is measured with a high gloss checker manufactured by Horiba Ltd., at an angle of incidence 60° and an angle of light reception of 60°, and "glossiness (%) (before wiping off)" is determined. The initial glossiness is preferably 85% or more for generation of luster in the appearance.

### [Measurement of glossiness (after wiping off): "glossiness (%) (after wiping off)"]

The test piece subjected to measurement according to the above Measurement of glossiness (before wiping off) is used, the surface thereof is wiped five times by a waste cloth plentifully impregnated with acetone, and again subjected to measurement of the glossiness, and "glossiness (%) (after wiping off)" is determined. The "rate of change in glossiness (%)" is determined by "Rate of change in glossiness (%)" = ("Glossiness after wiping off (%)" - "Glossiness before wiping off (%)")/"Glossiness before wiping off (%)" × 100. The glossiness after wear is preferably 85% or more from the viewpoint of durability. The rate of change in glossiness is more preferably -5.0 to 0.0%.

### [Evaluation of heat generation during procedures]

After sanding of a nail was carried out, any dust and oil on the surface of the nail were removed by a a nail-specific solvent (mainly containing ethanol). The nail was coated with a basecoat agent so that the wet thickness was about 100 µm. The coating was performed by a brush. Thereafter, the composition was cured with irradiation by a LED lamp for nails (rated voltage: 100 to 110 V, consumed power at 50 to 60 Hz: 36 W, wavelength: 350 to 400 nm) for 30 seconds. The same method was performed to sequentially coat the surface of the basecoat with a colorcoat and a topcoat, and these were sequentially cured under the same conditions. The colorcoat used was Super Color EX (color: pastel peach) manufactured by PREGEL, and the topcoat used was each composition. "Heat generation during procedures" was evaluated with respect to fingernails (10 fingers) of the hand of one person, according to the following evaluation criteria. "Good" or "Fair" is preferred in consideration of stress on a subject.

### <<Evaluation criteria>>

Good: no hotness felt
Fair: slightly warm
Poor: hot.

### [Measurement of degree of yellowness]

An alkali-free glass plate of 0.7 mm thickness × 100 mm width × 100 mm length was coated with the composition at a thickness of 150 µm, and the composition was cured by irradiation with ultraviolet light at a cumulative amount of light of 30 kJ/m². The degree of yellowness was measured by visible/ultraviolet spectroscopy. The transmittance was measured in the wavelength range of 800 nm to 300 nm, and the degree of yellowness was calculated according to JIS K 7373: 2006. The number n of tests is 3, and the average value is calculated and defined as "degree of yellowness". The degree of yellowness is preferably 3.0 or less in order to maintain transparency.

**[Table 2]**

| Test item | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|
| Surface gloss (before wiping off) | Good | Good | Good | Good | Good | Good | Good | Good | Good | Good |
| Surface gloss (after wiping off) | Good | Good | Good | Good | Good | Good | Good | Poor | Poor | Poor |
| Glossiness (before wiping off) | 90 | 90 | 88 | 90 | 90 | 90 | 90 | 88 | 90 | 90 |
| Glossiness (after wiping off) | 88 | 88 | 88 | 86 | 86 | 89 | 89 | 86 | 86 | 86 |
| Percentage of change in glossiness | -2.2 | -2.2 | 0.0 | -4.4 | -4.4 | -1.1 | -1.1 | -2.3 | -4.4 | -4.4 |
| Heat generation during procedures | Good | Good | Good | Fair | Good | Good | Good | Good | Good | Good |
| Degree of yellowness | 2.1 | 1.8 | 1.8 | 2.0 | 1.6 | 1.8 | 1.8 | 1.5 | 1.8 | 1.8 |

It was found from comparison between Examples 1 to 7 and Comparative Examples 1 to 3 that, although superiority or inferiority was not found in terms of the rate of change in glossiness and the degree of yellowness, the surface gloss after wiping off was lost in Comparative Examples 1 to 3 and was maintained in Examples 1 to 7. It was also found from comparison between Examples 1 to 5 and Comparative Example 3 that, in a case where the component (c-1) was used as the component (C) and also the content of the monofunctional (meth)acrylate contained as the component (D) was 50% by mass or less based on the total of the component (D), the surface gloss after wiping off was maintained.

### Industrial Applicability

The present invention can allow for use as a topcoat in procedures in the nail field, without scratching of any surface by external friction, impact, or the like.

The present application is based on Japanese Patent Application No. 2022-177271 filed on November 4, 2022, the disclosure of which is incorporated by reference in its entirety.

## Claims

1. A photocurable composition for a nail or an artificial nail, comprising the following components (A) to (E), wherein, in a case where the component (C) comprises a component (c-1), a monofunctional (meth)acrylate is not contained as the component (D) or a content of a monofunctional (meth)acrylate contained as the component (D) is 50% by mass or less based on a total of the component (D):
component (A): a urethane-modified (meth)acrylate oligomer;
component (B): a (meth)acrylate having a bisphenol backbone;
component (C): one or more (meth)acrylates selected from the group consisting of a component (c-1) and a component (c-2) (except for the component (A) and the component (B)):
component (c-1): a (meth)acrylate having an alicyclic hydrocarbon backbone; and
component (c-2): a (meth)acrylate having an isocyanurate backbone;
component (D): a (meth)acrylate except for components (A) to (C); and
component (E): a photoinitiator.

2. The photocurable composition for a nail or an artificial nail according to claim 1, comprising 30 parts by mass or more and 90 parts by mass or less of the component (B) based on 100 parts by mass of the component (A).

3. The photocurable composition for a nail or an artificial nail according to claim 1 or 2, wherein the component (D) is one or more selected from the group consisting of a monofunctional (meth)acrylate, a difunctional (meth)acrylate and a trifunctional (meth)acrylate.

4. The photocurable composition for a nail or an artificial nail according to claim 1, further comprising an ultraviolet absorber.

5. The photocurable composition for a nail or an artificial nail according to claim 4, wherein the ultraviolet absorber is a hydroxybenzophenone derivative.

6. The photocurable composition for a nail or an artificial nail according to claim 1 or 2, wherein the photocurable composition for a nail or an artificial nail is for topcoat layer formation.
